# EUROPEAN PATENT APPLICATION

(11) **EP 3 059 242 A1**
(43) Date of publication of application: **24.08.2016**
(21) Application number: 14853355.7
(22) Date of filing: 17.10.2014
(51) Int. Cl.: C07K 7/02, A61K 47/42

(54) **ALPHA HELIX CELL-PENETRATING PEPTIDE MULTIMER, PREPARATION METHOD THEREFOR AND USE THEREOF**

(30) Priority: 17.10.2013 KR 20130123709
(71) Applicant: Seoul National University R&DB Foundation, Gwanak-gu Seoul 08826 (KR)
(72) Inventor: YU, Jaehoon, Yongin-si Gyeonggi-do 446-858 (KR); LEE, Yan, Seoul 151-015 (KR); HYUN, Soonsil, Seoul 142-817 (KR); JANG, Sangmok, Seoul 151-835 (KR)
(74) Representative: Ladendorf, Oliver
(86) International application number: PCT/KR2014/009778
(87) International publication number: WO 2015/057009

(57) **Abstract**

The present invention relates to an α-helical cell-penetrating peptide multimer, a preparation method thereof and the use thereof, and more particularly, to a peptide multimer comprising a plurality of amphipathic peptides, a method for preparing the peptide multimer, a composition for preventing or treating HIV, which comprises the peptide multimer as an active ingredient, and a composition for intracellular delivery of a biologically active substance, which comprises the peptide multimer and the biologically active substance.

## Description

### TECHNICAL FIELD

The present invention relates to an α-helical cell-penetrating peptide multimer, a preparation method thereof and the use thereof, and more particularly, to a peptide multimer comprising a plurality of amphipathic peptides, a method for preparing the peptide multimer, a composition for preventing or treating HIV, which comprises the peptide multimer as an active ingredient, and a composition for intracellular delivery of a biologically active substance, which comprises the peptide multimer and the biologically active substance.

### BACKGROUND ART

Antimicrobial peptides (AMPs) are natural peptides that are produced from the primary immune response of a host in order to protect the host from externally invading pathogens. These mainly damage the cell membrane of invading pathogens to thereby control the invading pathogens. However, some evidences recently suggested that such antimicrobial peptides can control invading pathogens by a mechanism other than the mechanism by which the antimicrobial peptides damage the cell membrane. Namely, these antimicrobial peptides can control bacteria by binding to their target in the pathogens to block the function of the pathogens. Generally, antimicrobial peptides carry a positive charge which binds to the negative charge of DNA or RNA, suggesting that the DNA or RNA of pathogens has a potential as a target. It was shown that a mechanism is also possible in which antimicrobial peptides penetrate mitochondria that are organelles in the pathogens to induce cell death, thereby controlling the pathogens. This is because the surface of mitochondria is charged with negative ions.

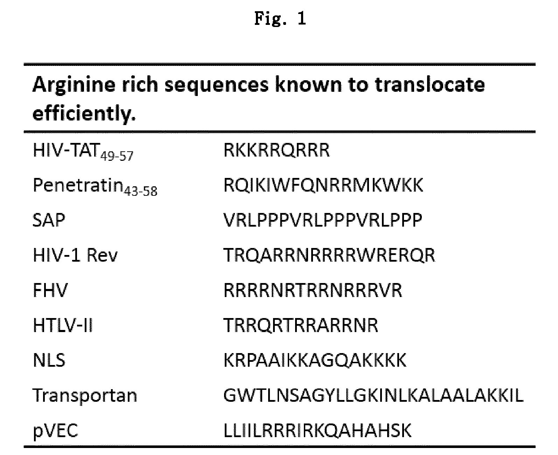

As suggested above, antimicrobial peptides do not kill pathogens by breaking the membrane of the pathogens, but can kill pathogens by binding to other targets in the cells. This is also demonstrated by the fact that cell-penetrating peptides (CPPs) having the ability to penetrate cells are distinguished from antimicrobial peptides capable of killing pathogens. Naturally occurring cell-penetrating peptides were found mainly in viruses. These peptides are peptides having cell-penetrating ability from cell-killing ability, because these should use all the functions of host cells for survival of viruses. Typical examples thereof include TAT peptide, Rev peptide and the like, which help viruses to penetrate cells without killing hosts. The penetratin peptide derived from the Antennapedia protein is a typical cell-penetrating peptide comprising 16 amino acids and having excellent cell-penetrating ability (Korean Patent No. 1095841). Like antimicrobial peptides, the penetratin peptide has a characteristic in that it is rich in positively charged arginine/lysine (FIG. 1).

The most distinct characteristic of the amino acid composition of cell-penetrating peptides is that it is rich in basic amino acids such as arginine and lysine. Because peptides comprising 7 or 9 consecutive arginine residues linked by amide bonds are also used as cell-penetrating peptides, such positive charges are essential for recognition of negatively charged cell surfaces. In addition, from the fact that arginine residues are more abundant than lysine residues, despite carrying the same positive charge, it can be seen that a guanidino group is a functional group that more easily cope with negative charges compared to a simple amino group.

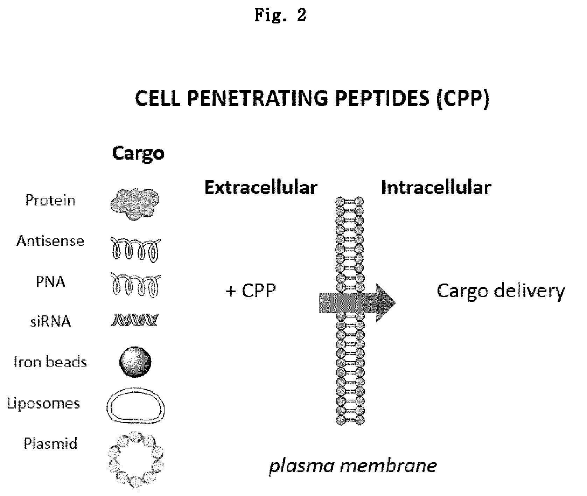

Such cell-penetrating peptides should have theoretically negligible cytotoxicity lower than that of antimicrobial peptides. Because of the low cytotoxicity of such cell-penetrating peptides, methods have been investigated which can connect many substances, which require intracellular delivery, to the cell-penetrating peptides in order to introduce such substances into cells. Oligonucleotides such as DNA or RNAi, or compounds (anticancer drugs) capable of causing biological changes or cytotoxicity, or specific proteins, may also be used as cargos that are linked to cell-penetrating peptides and introduced into cells (FIG. 2).

Mechanisms by which cell-penetrating peptides penetrate cells are largely classified into two methods, one of which is a method that involves direct penetration and endocytosis requiring heat. It is known that the mechanisms vary depending on the property of each cell-penetrating peptide or the cell-penetrating peptides enter cells using a combination of the two methods.

It may appear that a cell-penetrating peptide having cell-penetrating ability while having no cytotoxicity cannot be obtained, because the cell-penetrating ability and no cytotoxicity are contradictory conditions. Because of this fact, many cell-penetrating peptides also have the properties of antimicrobial peptides. Although cell-penetrating peptides are peptides having a maximized ability to enter cells without destroying the cells, the cell membrane can be cracked due to these cell-penetrating peptides, and positively charged cell-penetrating peptides can meet negatively charged intracellular substances such as DNA or RNA, thereby causing cytotoxicity.

The morphological characteristics of cell-penetrating peptides are similar to those of antimicrobial peptides. For penetration through the cell membrane, cell-penetrating peptides should have a positively charged hydrophilic group in order to recognize the negatively charged cell membrane, and should also be capable to form an α-helical shape in membrane conditions while recognizing hydrophobic molecules present in the cell membrane. For these reasons, cell-penetrating peptides and antimicrobial peptides should have amphipathic (hydrophobic and hydrophilic) properties. Thus, many cell-penetrating peptides are α-helical peptides, typical examples of which are α-helical cell-penetrating peptides such as penetratin and HIV Tat. However, such peptides can exhibit a desired cell-penetrating effect even when they are used at the lowest possible concentration (micromolar order), and thus there is a need for the development of a peptide that exhibits desired cell-penetrating ability even when being used at nanomolar concentrations.

Under this background, the present inventors have found that, when an amphipathic peptide comprising hydrophilic and hydrophobic amino acids comprises a linker at one or more specific amino acid positions, the cell penetrability of the peptide can be significantly increased, thereby completing the present invention.

### DISCLOSURE OF INVENTION

### TECHNICAL PROBLEM

It is an object of the present invention to provide a peptide multimer comprising α-helical cell-penetrating amphipathic peptides which can be efficiently delivered into cells while showing reduced cytotoxicity in cells, a preparation method thereof, and the use thereof for the prevention or treatment of HIV and the intracellular delivery of a biologically active substance.

### TECHNICAL SOLUTION

To achieve the above object, the present invention provides a peptide multimer comprising a plurality of homogeneous or heterogeneous α-helical amphipathic peptides.

The present invention also provides a method for preparing a peptide multimer, comprising the steps of:
constructing α-helical peptides comprising hydrophilic and hydrophobic amino acids;
selecting a plurality of homogeneous or heterogeneous α-helical peptides; and
connecting the plurality of selected α-helical peptides at one or more amino acid positions selected from the group consisting of i, i+3, i+4, i+7, i+8, i+10 and i+11 (where i is an integer).

The present invention also provides a composition for preventing or treating HIV, which comprises the above-described α-helical peptide multimer as an active ingredient.

The present invention also provides a composition for intracellular delivery of a biologically active substance, which comprises the peptide multimer of any one of claims 1 to 18 and the biologically active substance.

### BRIEF DESCRIPTION OF THE DRAWINGS

FIG. 1 is a list of known cell-penetrating peptides comprising a plurality of arginine or lysine residues.
FIG. 2 is a schematic view showing the mechanism by which cell-penetrating peptides are delivered into cells.
FIG. 3 shows amphipathic α-helical peptide monomers and dimers according to an embodiment of the present invention.
FIG. 4 shows the results of analyzing the cell-penetrating abilities of peptides according an embodiment of the present invention. Each error bar represents standard deviation (n = 3), and *** and n.s. indicate p<0.001 and no significant difference from control, respectively.
FIG. 4a shows FACS results for LK-1 (▲), LK-2 (■), LK-3 (●), LK-4 (◆) and R9 (▼) at various peptide concentrations after 12 hours of incubation with HeLa cells;
FIG. 4b shows a CLSM (confocal laser scanning microscopy) image of HeLa cells treated with FITC-labeled LK-3 (10 nM), in which the nucleus is stained with Hoechst 33442 (blue);
FIG. 4c shows the relative cell penetrability of peptides according to the present invention under an endocytosis inhibiting condition of 10 nM;
FIG. 4d shows the relative cell penetrability of peptides according to the present invention under an endocytosis inhibiting condition of 500 nM - control (white), wortmannin (gray), amiloride (dark gray) and 4°C (black).
FIG. 5 shows the results of measuring the amount of RNAi delivered into cells using peptides according to an embodiment of the present invention.
FIG. 6 shows confocal microscopy photographs indicating the intracellular delivery mechanisms of peptides according to an embodiment of the present invention (Ac in FIG. 6 means an acetyl group).
FIG. 6a shows a 500 nM FITC (fluorescein isothiocyanate)-labeled monomeric peptide (37°C, 24 hrs) (left) and a 500 nM FITC-labeled monomeric peptide treated with 50 *µ*g/mL wortmannin (37°C, 24 hrs) (right);
FIG. 6b shows a 500 nM Ac-FITC dimer (37°C, 24 hrs) (left) and a 500 nM Ao-FITC dimer treated with 50 *µ*g/mL wortmannin (37°C, 24 hrs) (right);
FIG. 6c shows a 500 nM FITC-labeled monomeric peptide treated with 15 *µ*g/mL amiloride (37°C, 24 hrs) (left) and a 500 nM FITC monomeric peptide (4°C, 2 hrs) (right);
FIG. 6d shows a 500 nM Ac-FITC dimeric peptide treated with 15 *µ*g/mL amiloride (37°C, 24 hrs) (left) and a 500 nM Ac-FITC dimeric peptide (4°C, 2 hrs) (right);
FIG. 6e shows a 500 nM Ac-dimal-FITC dimeric peptide (37°C, 24 hrs) (left) and a 500 nM Ac-dimal-FITC dimeric peptide treated with 50 *µ*g/mL wortmannin (37°C, 24 hrs) (right);
FIG. 6f shows a 500 nM Ac-dimal-FITC dimeric peptide treated with 15 *µ*g/mL amiloride (37°C, 24 hrs) (left) and a 500 nM Ac-dimal-FITC dimeric peptide (4°C, 2 hrs) (right).
FIG. 7 shows cytotoxicity test results for peptides according to an embodiment of the present invention.
FIG. 8 shows the inhibition of Tat-mediated transcription elongation by peptides according to the present invention in HeLa cells at peptide concentrations of 10 nM and 100 nM - control (white), 10 nM (gray) and 100 nM (dark gray):
   a) relative mRNA expression of TAR-luc/β-actin;
   b) relative mRNA expression of TAR-luc/18S rRNA;
   c) relative mRNA expression of TAR-luc/TAR.

   Each error bar represents standard deviation (n = 3), and (*), (**), (***) and n.s. are 0.01≤p<0.1, 0.001≤p<0.01, p<0.001, and no significant difference from control, respectively.

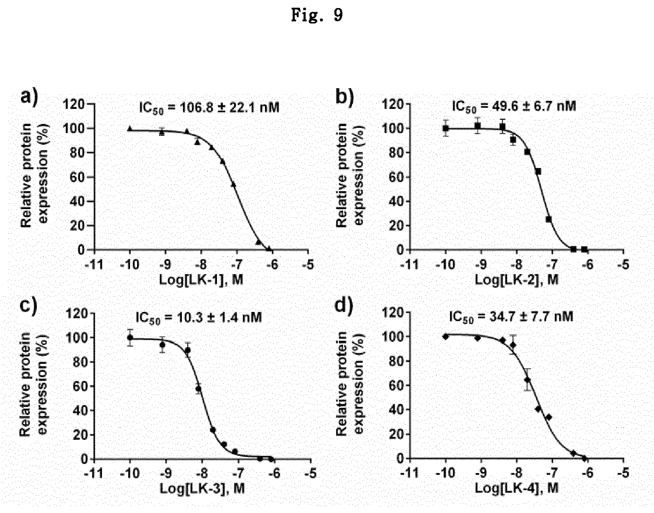

FIG. 9 shows the inhibition of luciferase activity by peptides according to the present invention in HeLa cells, and each error bar in FIG. 9 represents standard deviation (n = 3) :
   a) inhibition of luciferase activity by LK-1;
   b) inhibition of luciferase activity by LK-2;
   c) inhibition of luciferase activity by LK-3;
   d) inhibition of luciferase activity by LK-4.

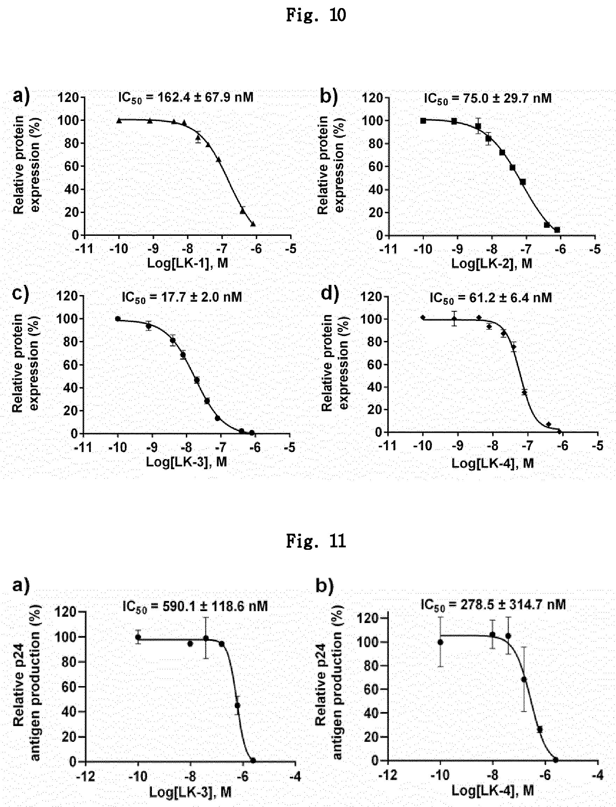

FIG. 10 shows the results of measuring the IC₅₀ values of LK peptides in RAW 264.7 cells.
FIG. 11 shows the inhibition of HIV-1 p24 antigen production by peptides according to the present invention in T-lymphoblastoid cells (MOLT-4/CCR5), and each error bar in FIG. 11 represents standard deviation (n = 3):
   a) inhibition of HIV-1 p24 antigen production by LK-3;
   b) inhibition of HIV-1 p24 antigen production by LK-4.

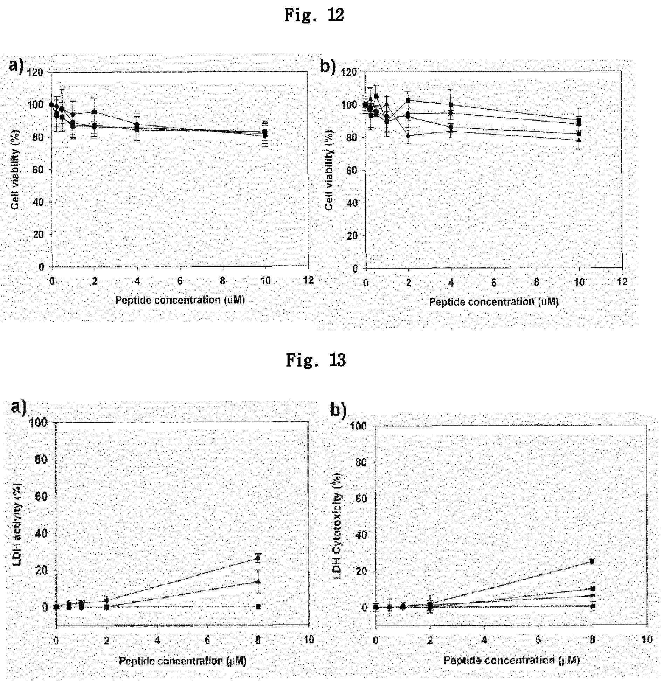

FIG. 12 shows the results of analyzing the cytotoxicity of peptides by an MTT assay:
   a) HeLa cells;
   b) RAW 264.7 cells.
FIG. 13 shows the results of an LDH assay performed to analyze the extent to which peptides destabilize the cell membrane:
   a) HeLa cells;
   b) RAW 264.7 cells.

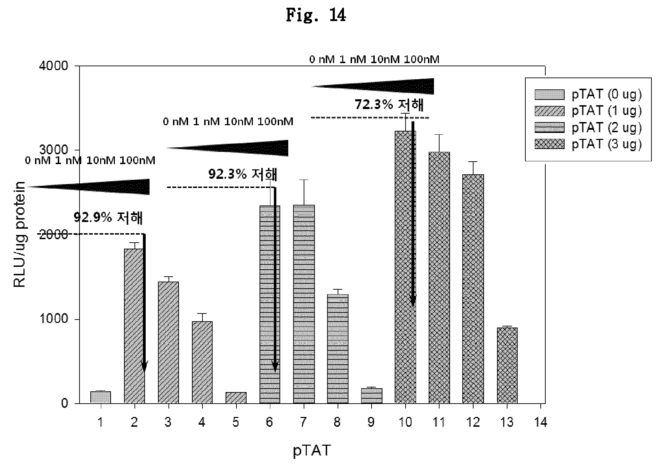

FIG. 14 shows the results of examining the reduction in the inhibitory ability of LK peptides according to the expression level of pTat.

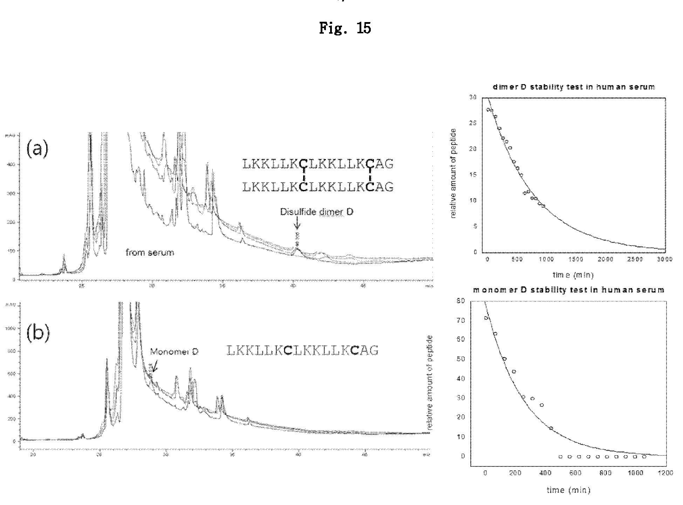

FIG. 15 shows the results of measuring the stabilities of a dimer D peptide and a monomer D peptide by HPLC: a) 20 M of disulfide dimer D in 25% human serum/RPMI 1640; b) 20 M of peptide 2 in 25% human serum/ RPMI 1640.

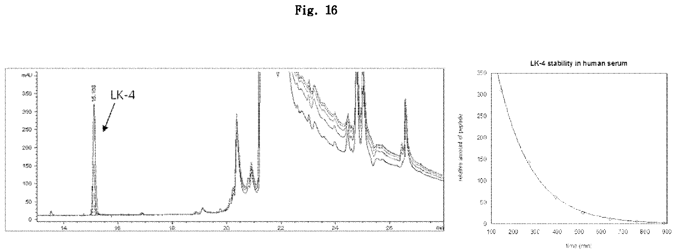

FIG. 16 shows the results of measuring the stability of an LK-4 peptide by HPLC: 20 M of LK-4 in 25% human serum/ RPMI 1640.

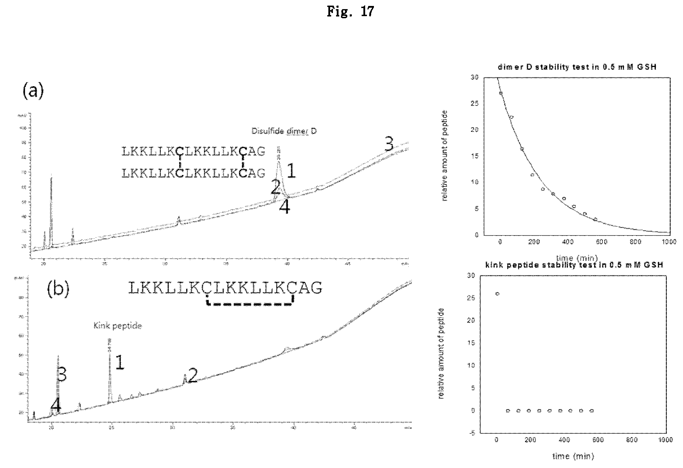

FIG. 17 shows the stabilities of peptide dimer D and kink D by HPLC in the presence of 0.5 mM GSH that is an *in vivo* reducing condition: a) 20M of disulfide dimer D in 0.5 mM reduced form of glutathione/ RPMI 1640. b) 20M of the kink peptide in 0.5 mM reduced form of glutathione/ RPMI 1640. Blue (1), 0 h; red (2), 5 h; green (3), 10 h; pink (4), 15 h.

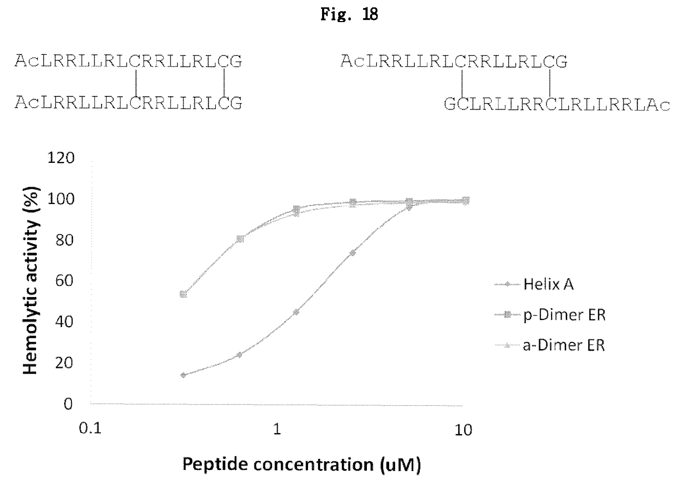

FIG. 18 shows the results of measuring hemolytic activity of peptide dimers connected in a parallel or antiparallel fashion.

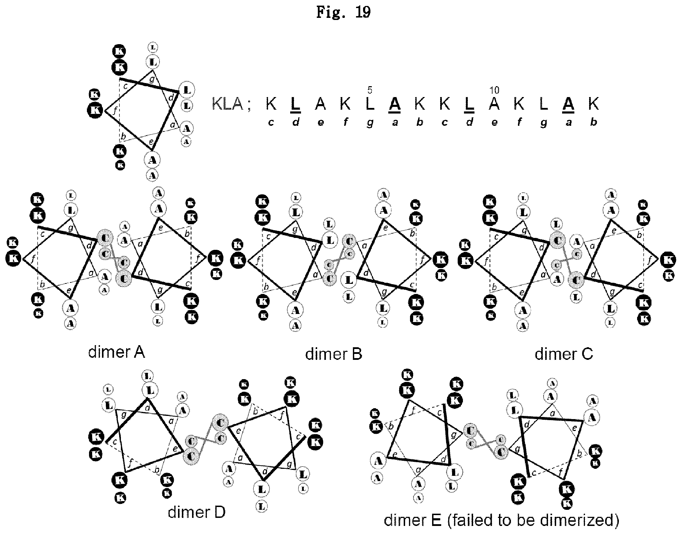

FIG. 19 is a helical wheel diagram showing structures in which some Leu residues in LK dimers are substituted with Ala.

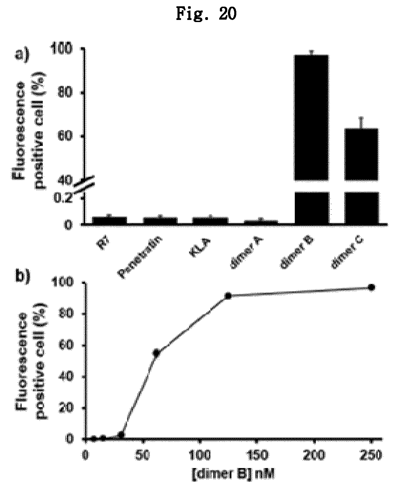

FIG. 20 shows the results of the cell-penetrating ability of the dimers shown in FIG. 19.

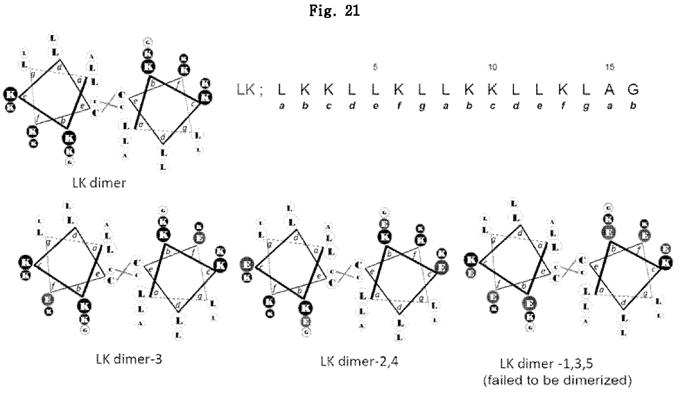

FIG. 21 is a helical wheel diagram showing peptide structures having a Lys-to-Glu substitution (K2, K4 → E2, E4).

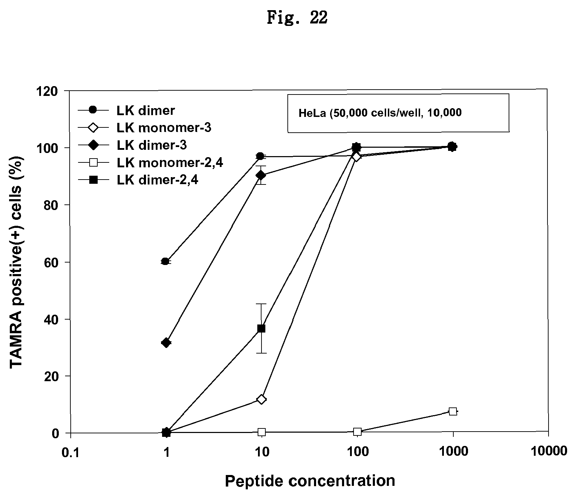

FIG. 22 shows the results of measuring the cell-penetrating abilities of the peptides of FIG. 21 for HeLa cells.

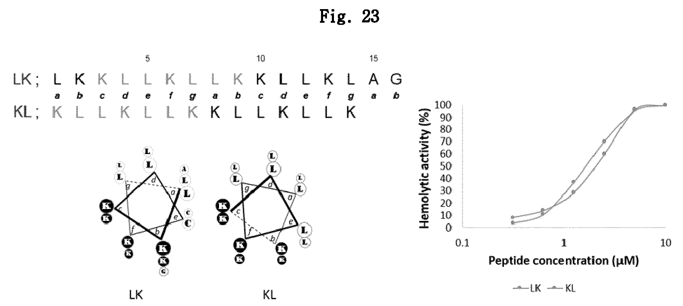

FIG. 23 shows the results of measuring the cell-penetrating abilities of peptides, which commonly contain SEQ ID NO: 1, for HeLa cells.

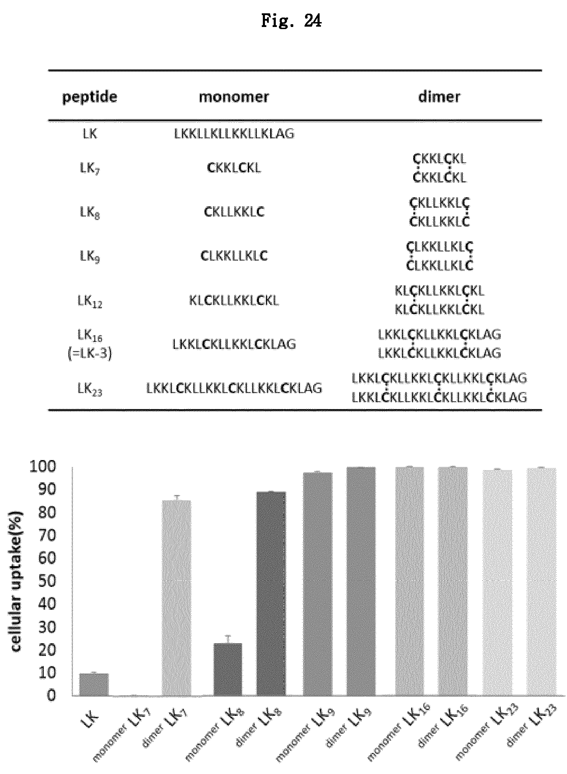

FIG. 24 shows the results of measuring the cell-penetrating abilities of peptides having various lengths for HeLa cells.

### BEST MODE FOR CARRYING OUT THE INVENTION

In one aspect, the present invention is directed to a peptide multimer comprising a plurality of homogeneous or heterogeneous α-helical amphipathic peptides.

The peptides in the present invention may be novel cell-penetrating peptides whose shape changes before and after intracellular delivery, and thus can have excellent cell-penetrating ability while showing reduced cytotoxicity in cells. For this, a method capable of maximally amplifying an α-helical shape present in cell-penetrating peptides, and a method that uses a special cytoplasmic environment such that this alpha-helix can easily disappear in the cytoplasm, are used.

Through the present invention, a method has been developed in which peptides having an artificially increased α-helical content are synthesized. When cells are treated with the synthesized peptides, the intracellular penetrability of the peptides will be increased due to the high α-helical content, and the alpha-helices will be removed in cells to minimize the toxicity of the peptides.

Conventional cell-penetrating peptides have a problem in that, because they have structural characteristics similar to those of antimicrobial peptides, they bind to their intracellular target to cause cytotoxicity when they penetrate cells. This is because the shape of cell-penetrating peptides before passage through the cell membrane is similar to their shape after their penetration into cells. Thus, the present inventors have peptides whose shape can greatly change before and after their penetration into cells.

Even α-helical peptides maintain a low α-helical content in a 100% aqueous solution in many cases, and most peptides have a low α-helical content of 50% or less in the aqueous cytoplasm. An amphipathic peptide according to the present invention or a dimer comprising the same maintains a high α-helical content in an extracellular environment, but the peptide structure can change in extracellular and intracellular environments under conditions in which the high α-helical content is broken in the cytoplasm. Namely, an on-off switch can be made in which the α-helical switch is turned-off in an extracellular environment or the cell membrane and turned-on in the cytoplasm. In this case, various cytotoxicities attributable to the alpha-helices of the peptides can be minimized in the cytoplasm. If a peptide has a high α-helical content, it will not be degraded by various peptidase, but a random shape will be made while being balanced with the α-helical shape, and the peptide having the random shape will be easily degraded by the action of a plurality of proteases, and thus the toxicity thereof can be minimized. Thus, when the α-helical content is maximized in an extracellular environment to maximize the cell-penetrating ability and is removed in the cytoplasm, cytotoxicity attributable to the alpha-helices can be minimized. A peptide satisfying such conditions is most suitable as a cell-penetrating peptide.

As one method for increasing cell-penetrating ability, the α-helical content of an amphipathic peptide can be increased. A method may be used in which the α-helical content is dramatically increased by covalently bonding the upper and lower branches of alpha-helices, and through this method, the cell-penetrating ability of the peptide can also be increased. Considering this, in order to increase the α-helical content, a plurality of amphipathic peptides may have a linker located at one or more amino acid positions selected from the group consisting of, for example, i, i+3, i+4, i+7, i+8, i+10 and i+11 (where i is an integer. The linker may preferably be located at two or more amino acid positions selected from the group consisting of i, i+3, i+4, i+7, i+8, i+10 and i+11 (where i is an integer). Herein, the plurality of amphipathic peptides may be connected in parallel to each other while maintaining the N-terminal to C-terminal direction. Alternatively, a portion of the plurality of peptides may be connected in the N-terminal to C-terminal direction, and the other portion may be connected in an antiparallel fashion in the C-terminal to N-terminal direction. It was shown that peptide multimers obtained by connecting peptides in a parallel or antiparallel fashion showed the same activity (FIG. 18). Through a peptide multimer comprising this linker, the α-helical content can be maintained.

In connection with this, Korean Patent Laid-Open Publication No. 2013-0057012 and US Patent Publication No. 2010-0292164 disclose that a disulfide bond is used to connect between two peptides. However, there was no example in which the α-helical content was increased by locating a linker at two or more amino acid positions to prepare a multimer, as described in the present invention. The α-helical content of the peptide in the peptide multimer according to the present invention may be 80%, preferably at least 85%, more preferably up to 100%, before cell penetration, for example, in a cell membrane condition in which trifluoroethanol and buffer are mixed at a ratio of 1:1.

In an embodiment, the amino acids of the peptide are not specifically limited as long as they can maintain the α-helical structure while showing amphipathic properties. For example, the hydrophilic amino acid may be one or more selected from the group consisting of arginine, lysine and histidine, and the hydrophobic amino acid may be one or more selected from the group consisting of leucine, valine, tryptophan, phenylalanine, tyrosine and isoleucine.

The peptide may comprise, for example, 5-50 amino acids, preferably 10-60 amino acids, more preferably 7-23 amino acids, which can form an α-helical structure that is a stable secondary structure. It can be seen that the peptides according to the present invention shows cell-penetrating ability dependent on their length. For example, it can be seen that peptides comprising less than 7 amino acids show significantly increased penetrating ability even at nanomolar concentrations, and particularly, peptides comprising 16-23 amino acids all show cell-penetrating ability at a concentration of 10 nM or lower (FIG. 24).

In order to collect the amine groups of the hydrophilic amino acids to one side of the α-helical peptide, one to three hydrophilic amino acids may be alternately arrayed, and the remaining sequence may comprise one to three alternately arrayed hydrophobic amino acids. For example, one to three hydrophilic amino acids may be arrayed alternately with one to three hydrophobic amino acids, and thus the amphipathic peptide may comprise a seven-amino-acid sequence in which at least one of the i+3 and i+4 positions of the amphipathic peptide has an amino acid having the same polarity as that at the i position. Preferably, the amphipathic peptide may comprise one or more of seven-amino-acid sequences in which one or two hydrophilic amino acids are arrayed alternately with one or two hydrophobic amino acids. Herein, if i is a hydrophilic amino acid, at least one of the i+3 and i+4 positions should be a hydrophilic amino acid, and if i is a hydrophilic amino acid, at least one of the i+3 and i+4 positions should be a hydrophobic amino acid.

The above sequence having seven amino acids may comprise, for example, one or more of amino acid sequences represented by the following formulas:

| | |
|---|---|
| XYXXYYX | YXYYXXY |
| XYYXYYX | YXXYXXY |
| XYYXXYX | YXXYYXY |
| XYYXXYY | YXXYYXX |
| XXYXXYY | YYXYYXX |
| XXYYXYY | YYXXYXX |
| XXYYXXY | YYXXYYX |

wherein X is a hydrophilic amino acid and Y is a hydrophobic amino acid.

Herein, the amphipathic peptide may contain hydrophobic amino acids capable of exhibiting the highest hydrophobicity, for example, one or more residues selected from the group consisting of leucine, tryptophan, valine, phenylalanine, tyrosine and isoleucine, in an amount of 25% or more, and the peptide can penetrate cells by hydrophobicity formed by these hydrophobic amino acids. The present inventors substituted the hydrophobic amino acid leucine (L) with alanine (A), and as a result, found that, if hydrophobic amino acids are not located at 25% or more of hydrophobic amino acids, the peptide does not show a desired cell-penetrating effect (FIGS. 19 and 20).

In addition, the amphipathic peptide may contain one or more positively charged hydrophilic amino acid residues selected from the group consisting of arginine, lysine and histidine, in an amount of 33% or more. If the amphipathic peptide contains positively charged hydrophilic amino acids in an amount of 33% or more, it can generally ensure a desired cell-penetrating ability. A peptide (in which positively charged amino acids are substituted with at least two negatively charged amino acids) containing positively charged hydrophilic amino acids in an amount of less than 33% (about 1/3 of hydrophilic amino acids) hardly penetrates cells even at a high concentration of 1 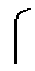 M, because it does not ensure net charges sufficient for cell penetration. The present inventors have found that, when lysine (K) residues 1 and 3 among positively charged lysine (K) residues are substituted with glutamic acid (K), the peptide containing positively charged hydrophilic amino acids in an amount of 33% or less did not show a desired cell-penetrating effect even at a concentration of 100 nM, but when the peptide was prepared into a dimer, the cell-penetrating ability could be increased about 100 times (FIGS. 21 and 22).

In one embodiment, the amphipathic peptide may comprise a sequence represented by the following SEQ ID NO: 11:
KLLKLLK (SEQ ID NO: 11).

Herein, amino acids of (LK)n may additionally be bound to the right end of the sequence of SEQ ID NO: 11, and amino acids of (LK)m may additionally be bound to the left end of the sequence of SEQ ID NO: 11, wherein n and m may be each independently an integer ranging from 0 to 2. Specifically, the amphipathic peptide may be the amino acid sequence LKKLLKLLKKLLKL represented by SEQ ID NO: 12 or the amino acid sequence KLLKLLKKLLKLLK represented by SEQ ID NO: 13.

The linker may comprise any bond that connects between peptides so as to exhibit the desired characteristics according to the present invention. For example, the linker may comprise a covalent bond. The covalent bond is not specifically limited as long as it is a covalent bond that can increase the α-helical content without inhibiting the function of peptides. For example, the covalent bond may be one or more selected from the group consisting of a disulfide bone between cysteines, a maleimide bond, an ester bond, a thioether bond, and a bond formed by a click reaction.

In order to maintain the stable alpha-helices of the peptide, it was attempted to bond the i position of the peptide with the i+3, i+4, i+7, i+8, i+10 or i+11 position using a covalent bond, and many types of linker compounds may be used for the bonding. However, although the α-helical content can be increased using such linker compounds, the linker compound and the peptide have a shortcoming in that they are not easily degraded in normal cytoplasmic conditions, because they form a more stable bond. If the alpha-helices are not broken, the peptide can show strong cytotoxicity. For this reason, a technology of aimlessly increasing the α-helical content should be excluded.

Considering this, the present inventors have found that a peptide multimer obtained by connecting amphipathic α-helical peptides by two or more disulfide bonds can form a strong bond to shRNA at nanomoles or less while having an α-helical content approaching 100%. This α-helical content is compared with the low α-helical content (about 10% in water) of a monomeric peptide, and the binding affinity thereof is also about 100-1000 times higher than that of the monomeric peptide. Through this high α-helical content, the cell-penetrating ability of the peptide can be increased, and the peptide can strongly bind to hairpin-shaped RNAi or shRNA, and thus can be an epoch-making structure for delivering RNAi into cells.

In the prior art, a method of increasing the α-helical content of peptides using artificially synthesized linker compounds was used, but in the present invention, a disulfide bond that can be obtained from cysteine can be used to increase the α-helical content of peptides and can also minimize the cytotoxicity of the peptides.

The present inventors have found that a multimer obtained by substituting the hydrophobic amino acids at the i position and i+7 position of peptides with cysteines and connecting the peptides through a disulfide bond shows a high α-helical content and also shows a strong affinity, which corresponds to kd values corresponding to nanomoles, for a hairpin-shaped RNA target.

If the amphipathic peptides are connected to each other by a disulfide bond, cysteines may be incorporated between the amino acids of the amphipathic peptides so that the peptides can be connected through the disulfide bond between the cysteines.

Herein, the peptide may comprise one or more of amino acid sequences represented by the following formulas:

| | |
|---|---|
| CYYXXYXCYYXXYXZW | (1) |
| XYYCXYXXYYCXYXZW | (2) |
| XYYXCYXXYYXCYXZW | (3) |
| XYYXXYCXYYXXYCZW | (4); and |
| XYYXXYXCYYXXYXCW | (5) |

wherein X, Z and W are hydrophobic amino acids, Y is a hydrophilic amino acid, and C is cysteine.

Specifically, the peptides may have at least one sequence selected from the group consisting of SEQ ID NOs: 1 to 10 shown in Table 1 below.

**Table 1**

| **SEQ ID NOs.** | **Sequences** |
|---|---|
| SEQ ID NO: 1 | CKKLLKLCKKLLKLAG |
| SEQ ID NO: 2 | LKKCLKLLKKCLKLAG |
| SEQ ID NO: 3 | LKKLCKLLKKLCKLAG |
| SEQ ID NO: 4 | LKKLLKCLKKLLKCAG |
| SEQ ID NO: 5 | LKKLLKLCKKLLKLCG |
| SEQ ID NO: 6 | CRRLLRLCRRLLRLAG |
| SEQ ID NO: 7 | LRRCLRLLRRCLRLAG |
| SEQ ID NO: 8 | LRRLCRLLRRLCRLAG |
| SEQ ID NO: 9 | LRRLLRCLRRLLRCAG |
| SEQ ID NO: 10 | LRRLLRLCRRLLRLCG |

Among the peptides satisfying the amino acid sequences shown in Table 1 above, the peptide according to the present invention may have an amino acid sequence represented by SEQ ID NO: 3 or 8.

The peptide having the amino acid sequence represented by SEQ ID NO: 3 or 8 comprises the hydrophobic amino acid leucine and the hydrophilic amino acid lysine or arginine. The peptide shows amphipathic properties by these hydrophilic and hydrophobic amino acids and has cell-penetrating ability by the α-helical structure.

The peptide multimer according to the present invention comprises homogeneous or heterogeneous α-helical peptides. It may be a homogeneous peptide multimer obtained by connecting a plurality of homogeneous peptides, or may be a heterogeneous peptide multimer comprising delivery peptides having excellent intracellular delivery ability and heterogeneous peptides capable of acting as a ligand for an intracellular target.

The multimer may be a form in which peptides having a plurality of functions are connected to each other while maintaining the desired functions of the peptides. For example, the multimer may be a dimer, a trimer, a tetramer or a pentamer. Preferably, it may be a dimer.

In another aspect, the present invention is directed to a method for preparing a peptide multimer, comprising the steps of: constructing α-helical peptides comprising hydrophilic and hydrophobic amino acids; selecting a plurality of homogeneous or heterogeneous α-helical peptides; and connecting the plurality of selected α-helical peptides at one or more amino acid positions selected from the group consisting of i, i+3, i+4, i+7, i+8, i+10 and i+11 (where i is an integer). Each of the elements according to the present invention as described above may likewise be applied to the method for preparing the peptide multimer.

In still another aspect, the present invention is directed to a composition for preventing or treating HIV, comprising the α-helical peptide multimer as an active ingredient. The present inventors have found that the peptide multimer according to the present invention shows a strong binding affinity for shRNA (short hairpin RNA) which is called TAR (trans-activating region) located in LTR (long terminal repeat) participating in efficient transcription of a genome introduced into the host of HIV-1 (Human immunodeficiency virus-1). In addition, the present inventors have found that a dimer formed by connecting the peptides having the sequence of SEQ ID NO: 3 by a cysteine-cysteine covalent bond contained therein also shows a very strong binding affinity for shRNA which is called TAR.

This correlation was further studied, and as a result, it was found that the peptide multimer according to the present invention can act particularly as an inhibitor of Tat-TAR interaction to inhibit HIV-1 replication. In addition, the peptide multimer according to the present invention has excellent cell-penetrating ability, and thus can act as an intracellular inhibitor against HIV-1 transcription.

Based on this fact, in still another aspect, the present invention is directed to a composition for preventing or treating HIV, comprising the α-helical cell-penetrating peptide multimer as an active ingredient.

The above-mentioned interaction between TAR RNA and viral Tat protein activates the transcription of viral genes. As a result, this interaction can become a target for development of substances for treating a disease caused by HIV-1. Even though this possibility is known, a pharmaceutical agent for inhibiting the binding of Tat to TAR RNA does not exist. This is because even low molecular compounds capable of penetrating cells cannot effectively bind to TAR RNA, and macromolecules hardly penetrate cells, even though they can inhibit the binding of Tat to TAR RNA.

Considering this, the present inventors have found that the α-helical cell-penetrating peptide multimer can penetrate cells to bind to TAR RNA, thereby directly inhibiting the binding between TAR RNA and Tat to thereby inhibit the transcription of HIV-1 genes.

The composition according to the present invention may be used for treatment of a HIV-infected patient or a patient who is actually or potentially exposed to HIV, but is not limited thereto. For example, the composition according to the present invention can be effectively used for treatment of HIV infection, after the patient was determined to be injected with HIV, that is, after the patient's blood was exposed to HIV during blood transfusion, organ transplantation, body fluid exchange, accidental needle sticking, or surgery.

In addition, the α-helical cell-penetrating peptide multimer according to the present invention may also be used as a peptide that recognizes the Bc12/Bax protein on the surface of mitochondria to induce the apoptosis of cancer cells.

The composition of the present invention may further comprise one or more pharmaceutically acceptable carriers. The pharmaceutically acceptable carriers should be compatible with the active ingredient, and may be one selected from among physiological saline, sterile water, Ringer's solution, buffered saline, dextrose solution, maltodextrin solution, glycerol, ethanol, and a mixture of two or more thereof. If necessary, the composition may contain other conventional additives such as an antioxidant, a buffer or a bacteriostatic agent. In addition, a diluent, a dispersing agent, a surfactant, a binder and a lubricant may additionally be added to the composition to prepare injectable formulations such as an aqueous solution, a suspension and an emulsion. Particularly, the composition is preferably provided as a lyophilized formulation. For the preparation of a lyophilized formulation, a conventional method known in the technical field to which the present invention pertains may be used, and a stabilizer for lyophilization may also be added. Furthermore, the composition can preferably be formulated according to diseases or components by a suitable method known in the art or by a method disclosed in Remington's Pharmaceutical Science, Mack Publishing Company, Easton PA.

The content of the active ingredient in the composition of the present invention and the method for administration of the composition can generally be determined by those skilled in the art based on the condition of the patient and the severity of the disease. In addition, the composition can be formulated in various forms, including powder, tablet, capsule, liquid, injectable solution, ointment and syrup formulations, and may be provided by use of a unit dosage form or multi-dosage container, for example, a sealed ampule or vial.

The composition of the present invention may be administered orally or parenterally. The composition according to the present invention may be administered, for example, orally, intravenously, intramuscularly, intraarterially, intramedullarily, intradually, intracardially, transdermally, subcutaneously, intraperitoneally, intrarectally, sublingually or topically, but is not limited thereto. The dose of the composition according to the present invention may vary depending on the patient's weight, age, sex, health condition and diet, the time of administration, the mode of administration, excretion rate, the severity of the disease, or the like, and can be easily determined by those skilled in the art. In addition, for clinical administration, the composition of the present invention may be prepared into a suitable formulation using a known technique.

In addition, the present invention is directed to a composition for intracellular delivery of a biologically active substance, which comprises the α-helical cell-penetrating peptide multimer and the biologically active substance binding to the peptide multimer.

The cell-penetrating ability of the peptide multimer according to the present invention can be at least 10 times higher than that of conventional cell-penetrating peptides. Specifically, conventional cell-penetrating peptides are used in at least micromolar concentrations to deliver a cargo into cells, whereas the cell-penetrating peptide multimer according to the present invention can ensure a desired cell-penetrating ability even when it is used at a concentration equal to about 1/10 of the minimum concentration of conventional cell-penetrating peptide used. Moreover, it was found that, if a biologically active substance, for example, an RNAi oligonucleotide molecule, is to be delivered into cells, the peptide multimer of the present invention shows a desired cell-penetrating ability even when it is used at a concentration in the nanomolar range.

As described above, according to the present invention, the cell-penetrating peptide multimer is used at a very low concentration, particularly, a concentration of several tens of nanomoles or less. Thus, even when a biologically active substance, for example, an RNAi oligonucleotide molecule, is used at a very low concentration compared to that used in the prior art, it can exhibit a desired effect. A low concentration of the cell-penetrating peptide and a low concentration of the biologically active substance can be sufficient conditions that can minimize cytotoxicity.

When the cell-penetrating peptide multimer according to the present invention penetrates the cytoplasm that is a reducing environment, the covalent bond in the multimer can be broken to form monomeric peptides. If the covalent bond in the peptide multimer continues to be maintained in cells, the peptide multimer can exhibit high cytotoxicity, because it generally has an excellent ability to bind to DNA or RNA. However, the peptide multimer whose covalent bond was broken in the cytoplasm can be easily hydrolyzed by many proteases in cells, because the chemical stability thereof significantly decreases while the α-helical content thereof also decreases rapidly.

Thus, the cell-penetrating peptide multimer according to the present invention exhibits an excellent ability to be delivered into cells, and can also achieve a desired effect even when a biologically active substance is used at a low concentration. In addition, it can be degraded in cells so that the cytotoxicity thereof can be minimized.

The biologically active substance, a kind of cargo, may be a substance that binds to the cellular transmembrane domain so as to be delivered to the cell to thereby regulate any physiological phenomena *in vivo.* For example, the biologically active substance may be DNA, RNA, siRNA, an aptamer, a protein, an antibody or a cytotoxic compound, but is not limited thereto.

In addition, a substance for regulating biological activity or function or other delivery carrier may additionally be bound to the peptide multimer according to the present invention. In this case, the peptide multimer and the substance for regulating biological activity or function or other delivery carrier can form a complex structure. The substance or delivery carrier may be connected to the multimer by, for example, a non-covalent bond or a covalent bond. The non-covalent bond may be one or more selected from the group consisting of, for example, a hydrogen bond, an electrostatic interaction, a hydrophobic interaction, a van der Waals interaction, a pi-pi interaction, and a cation-pi interaction. The covalent bond may be either a degradable bond or a non-degradable bond. The degradable bond may be a disulfide bond, an acid-degradable bond, an ester bond, an anhydride bond, a biodegradable bond, or an enzyme-degradable bond, but is not limited thereto. The non-degradable bond may be either an amide bond or a phosphate bond, but is not limited thereto.

The cytotoxic compound can be connected to the peptide multimer by a non-covalent bond such as an electrostatic bond or a host-guest bond. For example, the cytotoxic compound may be doxorubicin, Methotrexate, Paclitaxel, Cisplatin, Bleomycin, taxol, berberine or curcumin, but is not limited thereto. If the biologically active substance is a protein or an antibody, it may include any drug that binds to a certain target in a cell, and the multimer can be introduced by fusion to the N-terminus or C-terminus of the protein or antibody.

In some cases, methotrexate against cancer cells (MCF7) having drug resistance may be connected to the peptide multimer so that it can be used as a novel substance capable of destroying the cancer cells. Furthermore, a physiologically active small molecule (taxol, berberine, curcumin, etc.) that is hydrophobic in nature may be connected to the peptide multimer to increase the concentration at which it is delivered into cells. In addition, an antibody, a protein that is an antibody fragment, or a protein drug, may be connected to the dimeric peptide of the present invention and delivered into cells, and an oligonucleotide drug (siRNA, asDNA, DNA, or an aptamer) may also be connected to the dimeric peptide and delivered into cells.

### EXAMPLES

Hereinafter, the present invention will be described in further detail with reference to examples. It will be obvious to a person having ordinary skill in the art that these examples are illustrative purposes only and are not to be construed to limit the scope of the present invention. Thus, the substantial scope of the present invention will be defined by the appended claims and equivalents thereof.

### Example 1: Synthesis of Peptide Monomers and Dimers

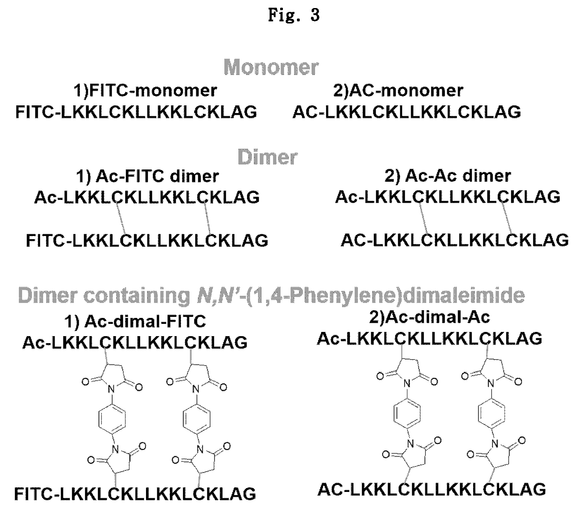

Monomeric LK(LKKLLKLLKKLLKLAG), monomeric A(CKKLLKLCKKLLKLAG), B(LKKCLKLLKKCLKLAG), C(LKKLCKLLKKLCKLAG), D(LKKLLKCLKKLLKCAG) and E(LKKLLKLCKKLLKLCG), each having two cysteine residues, a monomeric AR(LRRLLRLLRRLLRLAG) in which all the K residues in the amino acid sequence of LK are substituted with R, monomeric RA(CRRLLRLCRRLLRLAG), RB(LRRCLRLLRRCLRLAG), RC(LRRLCRLLRRLCRLAG), RD(LRRLLRCLRRLLRCAG) and RE(LRRLLRLCRRLLRLCG), each having two cysteine residues, etc., were synthesized using a solid-phase synthesis method and Fmoc chemistry. Dimeric peptides having disulfide attached thereto were obtained by oxidizing purified monomeric peptides under air oxidation conditions (dimeric A, B, C, D, E, RA, RB, RC, RD, and RE). As shown in FIG. 3, dimal peptides were synthesized according to the Michael reaction using a spacer.

In order to observe the property of penetrating cells, peptides having FITC labeled at the N-terminus thereof were also prepared. The molecular weights of the synthesized peptides were analyzed by a MALDI-TOF mass spectrometer as described below.

LK - MS [M+H]⁺: 1861.3 (calcd.), 1862.3 (found), FITC-labeled LK - MS [M+H]⁺: 2208.4 (calcd.), 2208.0 (found), C - MS [M+H]⁺: 1841.2 (calcd.), 1840.8 (found), FITC-labeled C - MS [M+H]⁺: 2188.2 (calcd.), 2188.5 (found)), dimer C - MS [M+H]⁺: 3677.4 (calcd.), 3677.9 (found), FITC-labeled dimer C - MS (M+H⁺): 4024.4 (calcd.), 4024.1 (found)), dimer MS [M+H]⁺: 2381.3 (calcd.), 2381.7 (found)), FITC-labeled dimer - MS [M+H]⁺: 4519.5 (calcd.), 4520.1 (found)).

### Example 2: CD (Circular Dichroism) Analysis of Synthesized Peptides

The secondary structures of the synthesized monomeric, dimeric or dimal peptides were observed using CD (circular dichroism). Particularly, the dimers showed an α-helical content approaching 100% even in an aqueous solution environment. This α-helical content very differs from that of the monomers (30% in an aqueous solution).

It is thought that when two disulfide bonds are present at i and i+7 positions in one direction of the alpha-helices, the two covalent bonds can maintain a high-alpha helical content by binding the α-helical shapes. Like the dimeric peptides, the dimal peptides also had a high alpha-content, because they were synthesized using two covalent bonds. However, the dimal peptides had an α-helical content lower than that of the dimeric peptides due to the softness of the molecule.

The dimeric/dimal peptides were treated with DTT that is a reducing environment (similar to the environment of the cytoplasm). Through this treatment, it is possible to observe how the dimeric/dimal peptides change in the cytoplasm that is a reducing environment. It was observed that the dimers were degraded into monomers, and the monomers had a significantly low α-helical content. However, the dimal peptides maintained the α-helical structure even when they were treated with DTT.

### Example 3: Cell Penetration Test for Peptides

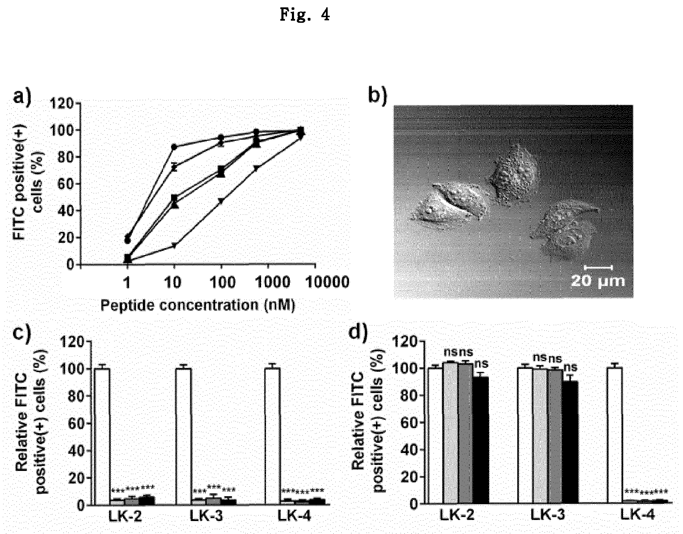

The intracellular uptakes of three different peptides were compared to one another by an FACS experiment at various concentrations of FITC-labeled peptides (FIG. 4). At a high peptide concentration (500 nM), the dimer (LK-3) and the monomer (LK-1 or LK-2) showed high uptake efficiencies (90% or more) with little or no difference. However, at a low concentration (10 nM), it was observed that the dimer (LK-3) showed high uptake efficiency, whereas the uptake efficiency of the monomer was greatly reduced to less than 10%.

Particularly, it was observed that the dimal peptide (LK-4) was uptaken even at a low concentration, but the uptake efficiency thereof was about 10-15% lower than that of the dimer (90% or more). It was shown that the cell-penetrating ability was higher in the order of dimer > dimal > monomer at both low concentration and high concentration.

### Example 4: siRNA Delivery Using Dimers

Using 10 dimeric peptides (dimeric A, B, C, D, E, RA, RB, RC, RD and RE) and control peptides, an experiment was performed to examine whether RNAi (Dy547-labeled) easily penetrates cells. As the control peptides, LK, Rev, monomeric C, kink C and kink D were used. A positive control containing 0.8 µL of DharmaFECT was used. The dimeric peptides were used at a concentration of 100 nM, and the monomeric peptides were used at a concentration of 200 nM, and RNAi was used at a concentration of 50 nM. Each of the peptides was mixed with RNAi to prepare mixture solutions. Each of the mixture solutions was added to HeLa cells (2.0 x 10³ cells/well) and incubated for 24 hours. Next, the cells were washed once with PBS, and then the amount of RNAi that penetrated the cells was observed with a fluorescent confocal microscope.

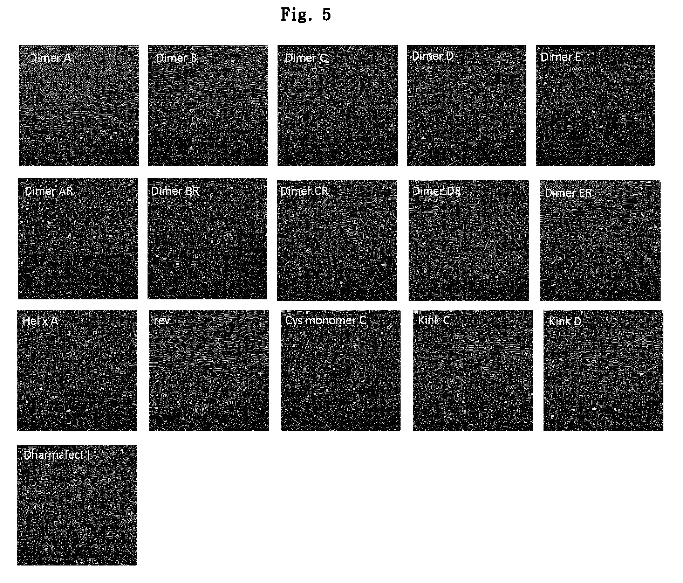

As can be seen in FIG. 5, the amount of RNAi delivered into the cells was larger in the order of dimer RE > dimer C > dimer D, but when the monomeric peptides were used, RNAi was not substantially delivered.

### Example 5: Analysis of Intracellular Penetration Mechanisms of Peptides

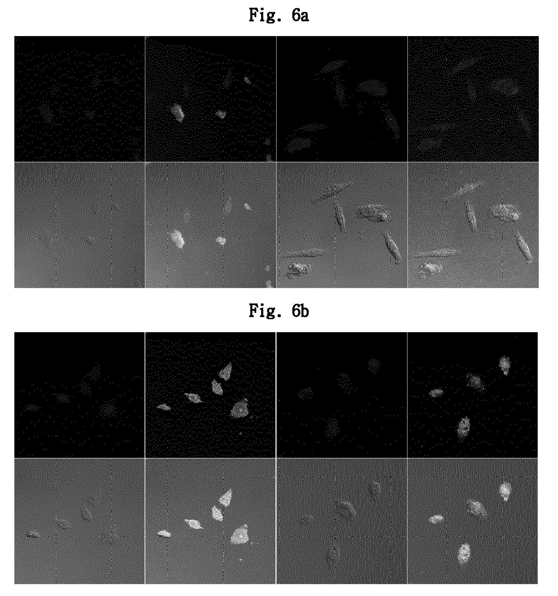

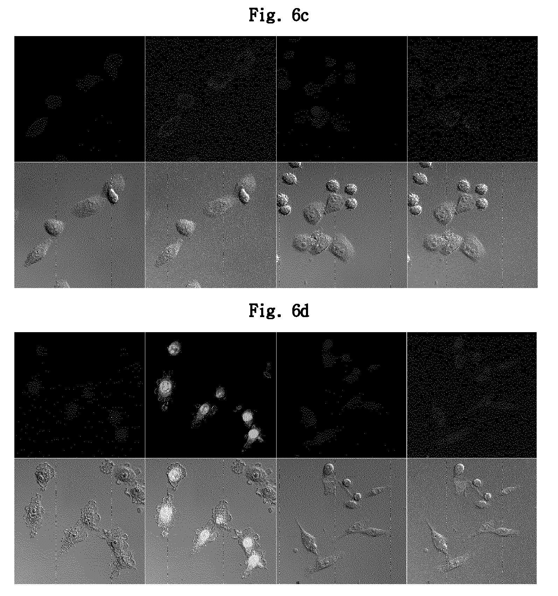

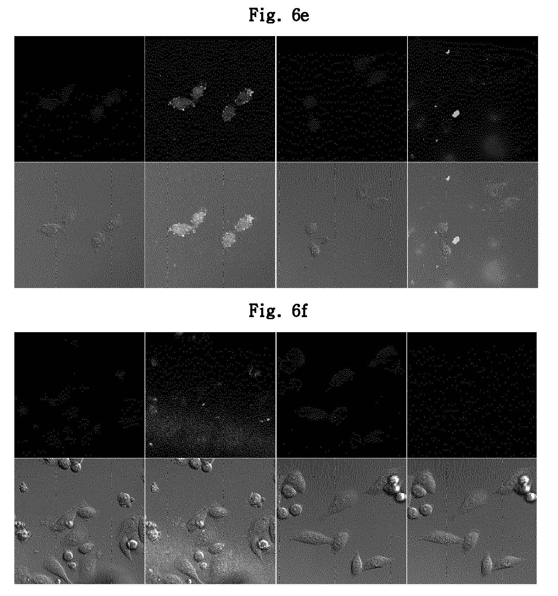

Uptake mechanisms of the peptides were compared using FITC-labeled peptides and confocal microscopy (FIG. 6).

The monomers were slightly delivered at a low temperature (ATP-independent uptake) or in a macropinocytosis inhibiting condition, suggesting that the monomers can penetrate cells by both an energy-independent mechanism and an energy-dependent mechanism. It can be obviously seen that the dimers also showed excellent cell-penetrating ability by the two mechanisms and had much more excellent cell-penetrating ability compared to the monomers. However, the dimal peptides had no cell-penetrating ability at low temperature and did not substantially enter the cells even in the macropinocytosis inhibiting condition, suggesting that the dimal peptides enter only the energy-dependent pathway. It can be seen that the monomers and the dimers enter cells by a direct method that is the energy-independent pathway, and cleavage of the disulfide bonds of the dimers plays an important role in the energy-independent pathway.

### Example 6: Cytotoxicity Test for Peptides

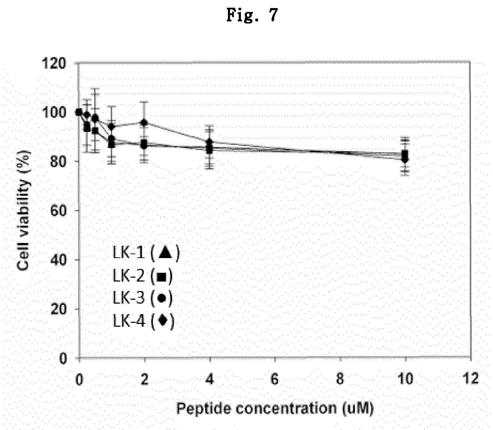

The cytotoxicity of the peptides was examined by treating cancer cells with the peptide and analyzing the activity of the cells by an MTT assay. As a result, all the three peptides (monomer, dimer and dimal) showed no cytotoxicity at a concentration of up to 5 µM (FIG. 7).

The present invention discloses a method for maximizing the α-helical content of an α-helical peptide having cell-penetrating ability and a peptide having increased delivery or penetration ability, synthesized by the method. To maximize the α-helical content, two thiols were attached to a monomeric peptide, and a dimeric peptide containing two disulfide bonds was prepared from the monomeric peptides using air oxidation conditions.

### Example 7: Measurement of Binding Affinity for TAR RNA and α-helical Content

Using the peptide having the sequence of SEQ ID NO: 3, the LK peptides shown in Table 2 below were prepared.

**Table 2**

| peptide | Sequences of peptide^{[b]} | *K*_{d} (nM) | α-helicity (%)^{[d]} |
|---|---|---|---|
| LK-1 | LKKL**L**KLLKKL**L**KLAG | 63^{[c]} | 24.4/77.4 |
| LK-2 | LKKL**C**KLLKKLCKLAG | 9.6^{[c]} | 27.9/77.1 |
| LK-3 | 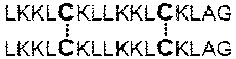 LKKLCKLLKKLCKLAG LKKLCKLLKKLCKLAG | 0.061^{[c]} | 90.6/99.0 |
| LK-4 | 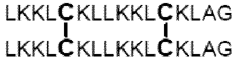 LKKLCKLLKKLCKLAG LKKLCKLLKKLCKLAG | 0.059 | 87.0/91.6 |
| R9 | RRRRRRRRR | n.d^{[e].} | 12.9/15.2 |

The amphipathic peptide dimer LK-3 contained two disulfide bonds in each chain and had an affinity of nanomoles or less for TAR RNA. Because the disulfide bonds in LK-3 can be degraded in the cytoplasmic environment, the reducible monomeric peptide LK-2 was used as a control. The non-reducible dimer LK-4 was composed of peptide chains connected by two N,N'-(1,4-phenylene)dimaleimide linkers. In Table 2 above, the disulfide bonds in LK-3 are indicated by dotted lines, and N,N-(1,4-phenylene)dimaleimide linkers in LK-4 are indicated by solid lines.

### (1) Binding Affinity

The *K*_{d} values (dissociation constants) of the peptides for binding to TAR RNA were measured by fluorescence anisotropy at 20°C using a rhodamine-Rev peptide as a probe. The results of the measurement indicated that the affinities of LK-3 and LK-4 were 100-1000 times higher than those of the monomeric peptides (LK-1 and LK-2).

### (2) α-helical Content (Alpha-Helicity)

Using CD (circular dichroism), the first alpha-helicity value was measured in a simulated membrane condition of PBS (pH 7.4), and the second value was measured in 50% TFE in the same buffer. The results of the measurement indicated that the alpha-helicity of the dimeric peptides was higher than that of the monomeric peptides. In fact, it was shown that LK-3 and LK-4 showed an alpha-helicity of about 90%.

### (3) Cell-Penetrating Ability

LK peptides labeled with FITC (fluorescein isothocyanate) were incubated with HeLa cells (human cervical cancer cell line) using the known cell-penetrating peptide R9 consisting of 9 arginine residues. The results of FACS (fluorescence activated cell sorting) analysis (FIG. 4) indicated that the percentage of FITC positive cells (cell-penetrating ability) was higher in the order of R9 < LK-1 and LK-2 < LK-4 < LK-3 at all the concentrations used in the analysis (FIG. 4a).

At a high concentration (>500 nM), the monomeric and dimeric peptides showed a cell penetration rate approaching 100%. However, the monomeric peptide showed a penetration efficiency of only 40% at a very low concentration (10 nM) and a penetration efficiency of only 70% at 100 nM, whereas the dimeric peptide had a cell penetration rate of 70-90%. The control R9 showed a very low cell penetration rate at 10 nM. As a result, it could be seen that the cell-penetrating ability of the LK peptides was greatly increased by formation of the dimer. In addition, it was shown that a considerable portion of the peptide dimer was delivered into the cell nucleus (FIG. 4b).

### (4) Examination of Intracellular Penetration Mechanism

In order to examine the mechanism used in intracellular penetration, LK peptides were tested under various endocytosis inhibiting conditions. Intracellular penetration of the LK peptides at a low concentration (10 nM) was almost completely inhibited by lowering the temperature to about 4°C or by treatment with an endocytosis inhibitor (wortmannin or amiloride). Thus, it can be seen that low concentrations of the LK peptides are internalized by an energy-dependent endocytic pathway and penetrated by macropinocytosis or clathrin-mediated endocytosis, even though the activities of the LK peptides significantly differ from these concentrations (FIG. 4c). At a high concentration (500 nM), all the LK peptides showed a cell uptake of >80%, and penetrated the cells according to various mechanisms. It can be seen that LK-4 (non-reducible dimer) entered the cells according to a mechanism similar to that at the low concentration (FIG. 4d). For example, the intracellular penetration of wortmannin or amiloride was not substantially inhibited by the monomer LK-2 and the dimer LK-3, and the internalization efficiency of these peptides did not change even at 4°C. Thus, it can be seen that LK-2 and LK-3 penetrate cells at a high concentration by other type of energy-dependent pathway which is neither the receptor-mediated endocytosis nor macropinocytosis.

Intracellular penetration of 500 nM of the non-reducible LK-4 dimer comprising maleimide linkers was completely inhibited by lowering the temperature or by treatment with the endocytosis inhibitor. From the above results, it can be seen that energy-dependent intracellular penetration of the peptide dimer at low concentrations is more promoted as the α-helical content decreases, whereas the monomeric peptide penetrates cells in an energy-independent fashion (for example, hole or carpet formation).

### (5) Examination of Effect on Inhibition of Tat-TAR Interaction

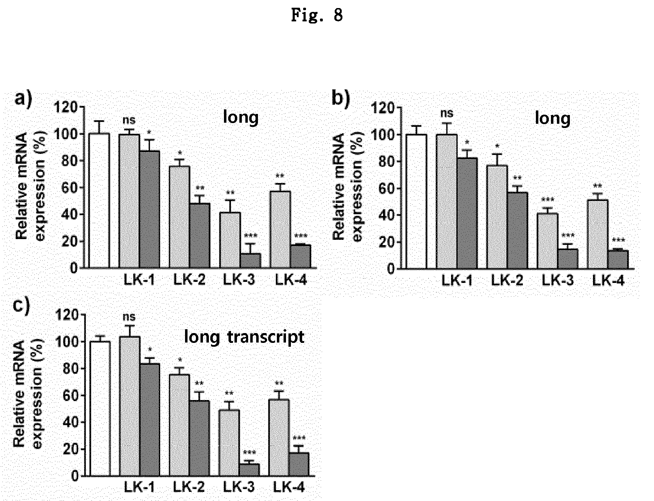

Because the LK dimeric peptide can be delivered into cells at nanomolar concentrations, whether the LK dimeric peptide can inhibit a viral target in host cells was examined. HeLa cells were transfected with a plasmid comprising pLTR-luc, HIV-1 LTR promoter and firefly luciferase gene together with a plasmid comprising pTat and HIV-1 Tat gene, thereby constructing a luciferase reporter system in the HeLa cells. In this system, the expressed Tat protein that is an anti-terminator interacts with TAR RNA under the LTR promoter to increase the transcription of the luciferase gene. The LK peptide and the reporter cells were incubated for 12 hours, and then the relative amount of mRNA was determined by RT-PCR (FIG. 8). As a result, in comparison with two housekeeping genes (β-actin and 18S rRNA), the mRNA level of the luciferase gene decreased in a manner dependent on the amount of the peptide (FIGS. 8a and 8b). In addition, in order to demonstrate that the LK peptide does not interfere with binding to the transfected plasmid DNA and transcription, the transcription of the long luciferase gene comprising the total TAR RNA transcribed was measured. It was shown that the ratio of TAR-luc (long transcript) to TAR (total transcript) was similar to the ratio of mRNA of TAR-luc to that of the housekeeping gene. This suggests that the Tat-TAR interaction is inhibited by the LK peptide at the transcription level (FIG. 8c).

Regarding the inhibition of Tat-mediated transcription, the monomers (LK-1 and LK-2) showed an inhibitory effect of less than 50% even at 100 nM, whereas the peptide dimers (LK-3 and LK-4) had a higher cell-penetrating ability while they showed an inhibitory activity of about 50% at 10 nM and an inhibitory activity of 80% at 100 nM. Because the abilities of LK-1 and LK-2 to penetrate HeLa cells were similar (FIG. 4a), it appears that the stronger inhibitory effect of LK-2 results from a stronger affinity for TAR RNA. Because the disulfide bond is easily degraded in the reducing cytoplasmic environment, LK-3 is reduced in the cytoplasm after internalization to form the monomer LK-2 that still binds to TAR RNA with a nanomolar affinity. Thus, the increased cell-penetrating ability of LK-3 is a main cause capable of inhibiting the Tat-TAR interaction at the transcription level.

### (6) Measurement of IC₅₀

The IC₅₀ values of the LK peptides were measured by a luciferase assay, and were lower in the order of LK-1 (107 nM) > LK-2 (49.6 nM) > LK-4 (34.7 nM) > LK-3 (10.3 nM) (FIG. 10). The IC₅₀ value of the dimer LK-3 was at least 10 times lower than that of the monomer LK-1, and this was thought to be because of the increased cell-penetrating ability and because when LK-3 was degraded in the cytoplasm, the concentration of the monomeric peptide increased twice or more. The IC₅₀ value of LK-3 was substantially identical to the dissociation constant of LK-2 (Kd = 9.6 nM), suggesting that penetration into the cell membrane no longer acted as a barrier against cell activity. Due to the specific construction that is not present in other peptide drugs, a great difference between the Kd and IC₅₀ values appeared.

FIG. 10 shows the results of measuring the IC₅₀ value of the LK peptides in RAW 264.7 cells (mouse monocyte/macrophage cell line) that are a similar cell-based measurement system. As can be seen in FIG. 10, the IC₅₀ value of the LK peptides was 15-150 nM.

### (7) Analysis of Inhibition of HIV-1 Replication and Cytotoxicity

Inhibition of HIV-1 replication in acute infected T-lymphoblastoid cells (MOLT-4/CCR5) was analyzed (FIG. 11). The IC₅₀ values of LK-3 and LK-4 in HIV-1 replication were 590.1 nM and 278.5 nM, respectively, whereas the IC₅₀ value of LK-1 was > 2 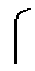 M. The IC₅₀ value greater than that in HeLa or RAW 264.7 cells could partially contribute to the reduction in the ability of the peptide to penetrate the T-lymphoblastoid cells. However, LK-3 showed no significant cytotoxicity for the host cells at a concentration of 2.56 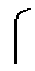 M or less, which is much higher than the IC₅₀ value for HIV-1 replication. It was shown that LK-4 had activity slightly higher than that of LK-3, but was cytotoxic for the host cells. This indicates that the LK dimers, particularly LK-3, can be used for the treatment of HIV-1.

The cytotoxicity of the peptide was analyzed by an MTT assay. The results of the analysis are shown in FIG. 12. When the MTT assay was performed at 24 hours after treatment with the peptide, it was shown that the peptide was not cytotoxic for both Hela cells and RAW 264.7 cells until it reached about 10 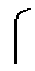 M.

### (8) Destabilization of Cell Membrane

The degree of destabilization of the cell membrane by the peptide was analyzed by an LDH assay. The results of the analysis are shown in FIG. 13. Referring to FIG. 13, when the LDH assay was performed at 12 hours after treatment with the peptide, it could be seen that the peptide did not substantially destabilize the cell membrane for both Hela cells and RAW 264.7 cells until it reached about 2 µM.

Because penetration of the peptide into the cell membrane occurred at a concentration of about 10 nM, it can be seen that intracellular penetration of the peptide has no direct connection with destabilization of the cell membrane.

It was shown that LK-3 (reducible dimer) and the monomer showed slight destabilization at 8 µM, whereas LK-4 (non-reducible dimer) showed little or no destabilization even at 8 µM.

### (9) Examination of Pattern of Inhibition of Tat-TAR Interaction

The reduction in the inhibitory ability of the LK peptides according to the expression level of pTat was analyzed. A Hela cell-based system was transfected with various amounts of a pTat plasmid, the inhibitory ability of the LK peptides was measured by luciferase. The results of the measurement are shown in FIG. 14. The details of each group in FIG. 14 are shown in Table 3 below.

**Table 3**

| Nos. | Details |
|---|---|
| 1 | pLTR(1µg)+pTAT(1µg)+lipofectamine(2µg) (12h) (48h) |
| 2 | pLTR(1µg)+pTAT(1µg)+lipofectamine(2µg)+10nM dimer(12h)(48h) |
| 3 | pLTR(1µg)+pTAT(1µg)+lipofectamine(2µg)+100nM dimer(12h)(48h) |
| 4 | pLTR(1µg)+pTAT(1µg)+lipofectamine(2µg)+10nM monomer(12h)(48h) |
| 5 | pLTR(1µg)+pTAT(1µg)+lipofectamine(2µg)+100nM monomer(12h)(48h) |
| 6 | pLTR(1µg)+pTAT(2µg)+lipofectamine(3µg) (12h) (48h) |
| 7 | pLTR(1µg)+pTAT(2µg)+lipofectamine(3µg)+10nM dimer(12h)(48h) |
| 8 | pLTR(1µg)+pTAT(2µg)+lipofectamine(3µg)+100nM dimer(12h)(48h) |
| 9 | pLTR(1µg)+pTAT(2µg)+lipofectamine(3µg)+10nM monomer(12h)(48h) |
| 10 | pLTR(1µg)+pTAT(2µg)+lipofectamine(3µg)+100nM monomer(12h)(48h) |
| 11 | pLTR(1µg)+pTAT(3µg)+lipofectamine(4µg) (12h) (48h) |
| 12 | pLTR(1µg)+pTAT(3µg)+lipofectamine(4µg)+10nM dimer(12h)(48h) |
| 13 | pLTR(1µg)+pTAT(3µg)+lipofectamine(4µg)+100nM dimer (12h) (48h) |
| 14 | pLTR(1µg) +pTAT (3 µg) +lipofectamine (4 µg) +10nM monomer(12h)(48h) |
| 15 | pLTR(1µg) +pTAT (3 µg) +lipofectamine (4 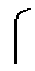 g)+100nM monomer(12h)(48h) |

| | |
|---|---|
| * dimer: LK-3, monomer: LK-2 | |

Referring to FIG. 14, it can be seen that the inhibitory ability of the LK peptides slowly decreased according to the expression level of pTat (92.9% > 92.3% > 72.3% (in the case of 100 nM LK-3). This indirectly suggests that competitive binding of Tat and the LK peptides has a deep connection with the expression of TAR-luciferase.

### (10) Peptide Stability

The stability of the peptide was measured by HPLC at 37°C. The half-life of the peptide was calculated by exponential decay modeling, and the results of the calculation are shown in Table 4 below and FIGS. 15 to 17 (rate constant plotting the peptide concentration in the time scale). In FIG. 14, the disulfide linkers in LK-3 are indicated by dotted lines, and the N,N'-(1,4-Phenylene)dimaleimide linkers in LK-4 are indicated by solid lines. The Kink peptide and LK-3 overlapped with various serum proteins. The life time was short, and the half-life was not determined in this condition. All the peptides introduced disappeared within 1 hour. The same results are also expected in dimer D.

**Table 4**

| Peptide | Sequences^{c} | Half life, T_{1/2} (h)^{b} | |
|---|---|---|---|
| | | In human serum | Reductive cytoplasmic condition |
| Monomer D | LKKLLKCLKKLLKCAG | 3 | N/A |
| Disulfide dimer D | LKKLLKCLKKLLKCAG LKKLLKCLKKLLKCAG | 9 | 3 |
| Kink peptide | 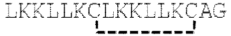 LKKLLKCLKKLLKCAG | NC^{d} | < 1^{e} |
| LK-3 | LKKLCKLLKKLCKLAG LKKLCKLLKKLCKLAG | NC^{d} | N/A^{f} |
| LK-4 | 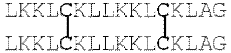 LKKLCKLLKKLCKLAG LKKLCKLLKKLCKLAG | 2 | N/A |

| | | | |
|---|---|---|---|
| NC: Not Calculable, N/A: not determined | | | |

The stabilities of peptide dimer D and monomer D in human serum *in vitro* were measured, and the results of the measurement are shown in FIG. 15. Referring to FIG. 15, it can be seen that dimer D similar to LK-3 showed a half-life of about 9 hours in 25% human serum, which is about three times longer than the half-life (3 hours) of the monomer thereof. This indicates that structural stabilization by formation of the disulfide dimer can inhibit degradation caused by protease present in serum.

The results of testing the stability of LK-4 in serum are shown in FIG. 16. The half-life of LK-4 comprising a bond different from a disulfide bond was measured to be 2 hours under the same conditions. Because LK-4 is not a dimer having a disulfide bond at the same position as that in dimer D, LK-4 cannot be compared directly with dimer D, but it can be seen that the stability of the peptide can change depending on the position at which the dimer is formed.

The results of testing the stabilities of peptide dimer D and kink D in 0.5 mM GSH that is an *in vivo* reductive condition are shown in FIG. 17. As an example that directly shows a structural effect on the stability of the peptide, when the dimer was formed so that the disulfide bond was stably located inside the structure, the time taken for the dimer to be reduced increased under the same condition. However, it was shown that when the disulfide bond was formed in the molecule so that it was relatively exposed, the half-life was within 1 hour.

Any person skilled in the art will appreciate that various applications and modifications based on the disclosure of the present invention are possible without departing from the scope of the present invention.

### INDUSTRIAL APPLICABILITY

As described above, the peptide multimer of the present invention, which comprises a linker located at one or more amino acid positions of a plurality of α-helical amphipathic peptides, can have high cell-penetrating ability, because it has a significantly increased α-helical content. Due to this excellent cell-penetrating ability, the peptide multimer can effectively deliver a variety of biologically active substances into cells, and the cytotoxicity thereof can be minimized after intracellular penetration. Thus, the peptide multimer of the present invention can be effectively used as an agent for preventing or treating diseases.

## Claims

1. A peptide multimer comprising a plurality of homogeneous or heterogeneous α-helical amphipathic peptides.

2. The peptide multimer of claim 1, wherein further comprising a linker located at one or more amino acid positions selected from the group consisting of i, i+3, i+4, i+7, i+8, i+10 and i+11 (where i is an integer).

3. The peptide multimer of claim 2, wherein the linker is located at two or more amino acid positions selected from the group consisting of i, i+3, i+4, i+7, i+8, i+10 and i+11 (where i is an integer).

4. The peptide multimer of claim 1, wherein the amphipathic peptide comprises one or more hydrophilic amino acids selected from the group consisting of arginine, lysine and histidine

5. The peptide multimer of claim 1, wherein the amphipathic peptide comprises one or more hydrophobic amino acids selected from the group consisting of leucine, valine, tryptophan, phenylalanine, tyrosine and isoleucine.

6. The peptide multimer of claim 1, wherein the peptide comprises 5-50 amino acids.

7. The peptide multimer of claim 1, wherein the peptide comprises 7-23 amino acids.

8. The peptide multimer of claim 1, wherein one to three hydrophilic amino acids and hydrophobic amino acids are arrayed respectively in the peptide.

9. The peptide multimer of claim 1, wherein the amphipathic peptide comprises one or more of seven amino acid sequences represented by the following formulas:
| | |
|---|---|
| XYXXYYX | YXYYXXY |
| XYYXYYX | YXXYXXY |
| XYYXXYX | YXXYYXY |
| XYYXXYY | YXXYYXX |
| XXYXXYY | YYXYYXX |
| XXYYXYY | YYXXYXX |
| XXYYXXY | YYXXYYX |
wherein X is a hydrophilic amino acid and Y is a hydrophobic amino acid.

10. The peptide multimer of claim 1, wherein the hydrophilic amino acid in the amphipathic peptide comprises one or more positively charged amino acid residue selected from the group consisting of arginine, lysine and histidine, in an amount of 33% or more.

11. The peptide multimer of claim 1, wherein the hydrophobic amino acid of the amphipathic peptide comprises one or more residues selected from the group consisting of leucine, tryptophan, valine, phenylalanine, tyrosine and isoleucine, in an amount of 25% or more.

12. The peptide multimer of claim 1, wherein the amphipathic peptide comprises a sequence represented by the following SEQ ID NO: 11:
KLLKLLK (SEQ ID NO: 11).

13. The peptide multimer of claim 1, wherein the peptide comprises one or more of amino acid sequences represented by the following formulas:
| | |
|---|---|
| CYYXXYXCYYXXYXZW | (1) |
| XYYCXYXXYYCXYXZW | (2) |
| XYYXCYXXYYXCYXZW | (3) |
| XYYXXYCXYYXXYCZW | (4); and |
| XYYXXYXCYYXXYXCW | (5) |
wherein X, Z and W are hydrophobic amino acids, Y is a hydrophilic amino acid, and C is cysteine.

14. The peptide multimer of claim 13, wherein the peptide comprises at least one sequence selected from the group consisting of SEQ ID NOs: 1 to 10.

15. The peptide multimer of claim 1, wherein the α-helical content of the peptide is at least 80% in a cell membrane condition in which trifluoroethanol and buffer are mixed at a ratio of 1:1.

16. The peptide multimer of claim 1, wherein the linker comprises a covalent bond that connects between peptides.

17. The peptide multimer of claim 16, wherein the covalent bond is at least one selected from the group consisting of a disulfide bone between cysteines, a maleimide bond, an ester bond, a thioether bond, and a bond formed by a click reaction.

18. The peptide multimer of claim 1, wherein the multimer is a dimer, a trimer, or a tetramer.

19. A method for preparing a peptide multimer, comprising the steps of:
constructing α-helical peptides comprising hydrophilic and hydrophobic amino acids;
selecting a plurality of homogeneous or heterogeneous α-helical peptides; and
connecting the plurality of selected α-helical peptides at one or more amino acid positions selected from the group consisting of i, i+3, i+4, i+7, i+8, i+10 and i+11 (where i is an integer).

20. The method of claim 19, wherein the plurality of selected α-helical peptides is connected to each other at two or more amino acid positions selected from the group consisting of i, i+3, i+4, i+7, i+8, i+10 and i+11 (where i is an integer).

21. The method of claim 19, wherein the plurality of α-helical peptides is connected to each other by a covalent bond that connects between peptides.

22. The method of claim 21, wherein the covalent bond is at least one selected from the group consisting of a disulfide bone between cysteines, a maleimide bond, an ester bond, a thioether bond, and a bond formed by a click reaction.

23. A composition for preventing or treating HIV, which comprises the α-helical peptide multimer of any one of claims 1 to 18 as an active ingredient

24. The composition of claim 23, wherein the peptide multimer binds to the TAR (trans-activating region) of HIV.

25. A composition for intracellular delivery of a biologically active substance, which comprises the peptide multimer of any one of claims 1 to 18 and the biologically active substance.

26. The composition of claim 24, where the biologically active substance is DNA, RNA, siRNA, an aptamer, a protein, an antibody or a low molecular compound.


<110>     Snu R&DB Foundation

<120>     Multimeric Cell Penetrating alpha-Helix Peptides, Method of
          Producing the Same And Use Thereof

<130>     PP-B1418

<150>     KR 10-2013-0123709
<151>     2013-10-17

<160>     13

<170>     KopatentIn 2.0

<210>     1
<211>     16
<212>     PRT
<213>     Artificial Sequence

<220>
<223>     Cell Penetrating Peptides

<400>     1
Cys Lys Lys Leu Leu Lys Leu Cys Lys Lys Leu Leu Lys Leu Ala Gly
  1               5                  10                  15

<210>     2
<211>     16
<212>     PRT
<213>     Artificial Sequence

<220>
<223>     Cell Penetrating Peptides

<400>     2
Leu Lys Lys Cys Leu Lys Leu Leu Lys Lys Cys Leu Lys Leu Ala Gly
  1               5                  10                  15

<210>     3
<211>     16
<212>     PRT
<213>     Artificial Sequence

<220>
<223>     Cell Penetrating Peptides

<400>     3
Leu Lys Lys Leu Cys Lys Leu Leu Lys Lys Leu Cys Lys Leu Ala Gly
  1               5                  10                  15

<210>     4

<211>    16
<212>    PRT
<213>    Artificial Sequence

<220>
<223>    Cell Penetrating Peptides


<400>    4
Leu Lys Lys Leu Leu Lys Cys Leu Lys Lys Leu Leu Lys Cys Ala Gly
1               5                   10                  15




<210>    5
<211>    16
<212>    PRT
<213>    Artificial Sequence

<220>
<223>    Cell Penetrating Peptides


<400>    5
Leu Lys Lys Leu Leu Lys Leu Cys Lys Lys Leu Leu Lys Leu Cys Gly
1               5                   10                  15




<210>    6
<211>    16
<212>    PRT
<213>    Artificial Sequence

<220>
<223>    Cell Penetrating Peptides


<400>    6
Cys Arg Arg Leu Leu Arg Leu Cys Arg Arg Leu Leu Arg Leu Ala Gly
1               5                   10                  15




<210>    7
<211>    16
<212>    PRT
<213>    Artificial Sequence

<220>
<223>    Cell Penetrating Peptides


<400>    7
Leu Arg Arg Cys Leu Arg Leu Leu Arg Arg Cys Leu Arg Leu Ala Gly
1               5                   10                  15

<210>    8
<211>    16
<212>    PRT
<213>    Artificial Sequence

<220>
<223>    Cell Penetrating Peptides


<400>    8
Leu Arg Arg Leu Cys Arg Leu Leu Arg Arg Leu Cys Arg Leu Ala Gly
1               5                   10                  15




<210>    9
<211>    16
<212>    PRT
<213>    Artificial Sequence

<220>
<223>    Cell Penetrating Peptides


<400>    9
Leu Arg Arg Leu Leu Arg Cys Leu Arg Arg Leu Leu Arg Cys Ala Gly
1               5                   10                  15




<210>    10
<211>    16
<212>    PRT
<213>    Artificial Sequence

<220>
<223>    Cell Penetrating Peptides


<400>    10
Leu Arg Arg Leu Leu Arg Leu Cys Arg Arg Leu Leu Arg Leu Cys Gly
1               5                   10                  15




<210>    11
<211>    7
<212>    PRT
<213>    Artificial Sequence

<220>
<223>    Cell Penetrating Peptides


<400>    11
Lys Leu Leu Lys Leu Leu Lys
1               5


<210>    12

<211>    14
<212>    PRT
<213>    Artificial Sequence

<220>
<223>    Cell Penetrating Peptides


<400>    12
Leu Lys Lys Leu Leu Lys Leu Leu Lys Lys Leu Leu Lys Leu
1               5                   10


<210>    13
<211>    14
<212>    PRT
<213>    Artificial Sequence

<220>
<223>    Cell Penetrating Peptides


<400>    13
Lys Leu Leu Lys Leu Leu Lys Lys Leu Leu Lys Leu Leu Lys
1               5                   10